# EUROPEAN PATENT APPLICATION

(11) **EP 2 660 592 A2**
(43) Date of publication of application: **06.11.2013**
(21) Application number: 11852487.5
(22) Date of filing: 28.12.2011
(51) Int. Cl.: G01N 33/02, C07K 16/16

(54) **METHOD FOR SELECTING CEREAL SEEDS SUITABLE FOR CONSUMPTION BY COELIAC PATIENTS**

(30) Priority: 28.12.2010 ES
(71) Applicant: Universidad de Sevilla, 41013 Sevilla (ES)
(72) Inventor: SOUSA MARTÍIN, Carolina, E-41012 Sevilla (ES); COMINO MONTILLA, Isabel, E-41012 Sevilla (ES); REAL CALDERÓN, Ana, E-41012 Sevilla (ES)
(74) Representative: Pons Ariño, Angel
(86) International application number: PCT/ES2011/000378
(87) International publication number: WO 2012/089867

(57) **Abstract**

The present invention relates to a simple method for selecting varieties of seeds from wheat, barley, rye and, in particular, oats, which enables said seeds, whether or not genetically manipulated, to be used in the production of gluten-free foods and beverages suitable for coeliac patients, and also the preparation of excipients for specific drugs. This invention is based on a simple immunological detection system that makes it possible to quantify the most immunogenic peptides of gluten with a view to ascertaining whether the cereal variety, preferably oats, can be used to produce suitable seeds that can form part of the diet of a coeliac patient. Preferred tests would make use of at least one antibody that recognizes the most immunogenic part of prolamines, the reactivity of which antibody vis-à-vis cereal seeds having been demonstrated to be proportional to the immunogenicity thereof with T-cells from coeliac

## Description

### FIELD OF THE INVENTION

The present invention relates to a simple system for immunological detection to quantify the immunogenic gluten peptides in order to know whether a cereal variety, preferably oat, can be used to produce suitable seed which may form part of the diet of a celiac. It is based on immunological assays with monoclonal antibodies that detect the 33-mer peptide from gliadin and other related peptides toxic to celiac patients.

This is a specific and reliable method that can be industrially applied to identify oat seeds and other grains that are tolerable and safe for celiac patients. Selected cereals can also be used to make ingredients that are part of drug carriers, and other industrial applications in products that will be eaten by the celiac collective. Therefore, the process described in this patent may eventually have a use not only in the food industry but also in the pharmaceutical sector, being the agricultural sector the preferred interest.

### BACKGROUND OF THE INVENTION

Celiac disease (CD) is defined as an alteration of the proximal small intestine mucosa associated with a permanent intolerance to gluten in genetically predisposed individuals, in which environmental factors are added. In this disease morphological changes of the intestinal mucosa are developed, with elongation of crypts and full or partial atrophy of intestinal villi after ingestion of cereal products, including those derived from wheat, barley, rye and possibly oats.

The clinical features of CD differ significantly depending on the age of presentation. Intestinal symptoms and growth delay are common in all children who have been diagnosed within the first years of their life (Stern et al., 2001, Eur J Gastroenterol Hepatol., 13:741-747). The development of the disease in later moments of childhood is marked by the onset of extra intestinal symptoms, such as refractory iron deficiency anemia, dyspepsia, constipation, bone and joint pain, ataxia, elevated liver enzymes, impaired reproductive sphere, etc. (Stern et al., 2001, Eur J Gastroenterol Hepatol., 13:741-747). CD is associated with autoimmune disorders such as Addison's disease, autoimmune thyroiditis, autoimmune hepatitis, etc. There are other diseases associated with celiac disease such as Down syndrome, Turner syndrome, Williams syndrome, IgA deficiency and neurological, endocrine, hepatic, musculoskeletal, cardiac, skin diseasesetc., including malignant tumors in different locations, mainly digestives.

Originally considered a rare malabsorption syndrome in children, today is considered a common disease that affects approximately 1% of the population, which can be diagnosed at any age and that affects many organ systems. Its genetic basis justifies the existence of more than one celiac patient within the same family.

Up to date, the only existing effective therapy is complete and permanent exclusion of gluten from the diet, which achieves the improvement or disappearance of symptoms in almost all patients. Although at first glance following a gluten free diet may seem simple, the fact is that certain situations favor involuntary ingestion as cereals like wheat are entrenched in our society, being present in a large number of manufactured products. Several studies have been carried out to determine the maximum amount of gluten that can be consumed daily for celiac patient safely. One study suggests that ingestion of gluten traces must be kept below 50 mg per day for celiac patients (Catassi et al., 2007, Am J Clin Nutr., 85:160-166). However, since there are different levels of tolerance to gluten intake among celiac patients, the amount of gluten that can be ingested by a celiac patient cannot be universally established, existing celiac patients who present alterations in the intestinal mucosa after eating only 10 mg of gluten daily (Catassi et al., 2007, Am J Clin Nutr., 85:160-166). Therefore, maintaining a gluten-free diet is not an easy task, not only because of the high cost involved, but also because the gluten is present in most foods that exist today on the market. For example, the gluten is a common ingredient in the human diet, perhaps after sugar is the food ingredient most used in Western civilization. On the other hand, it should be noted that, according to the Codex Alimentarius Commission, currently a "gluten-free" food is not one whose gluten content is 0, but containing an amount of gluten of less than 20 mg/kg . Therefore, although celiac patients follow a strict gluten-free diet, would involuntarily be consuming small amounts of it.

Gluten is a complex mixture of polypeptides present in cereals such as wheat, barley, rye and oats. The main immunogenic components of gluten belong to a family of proteins characterized by their high content of proline and glutamine residues, known as prolamins. The prolamins from wheat, barley, rye and oats, are called gliadins, hordeins, secalins and avenins, respectively. The disease is triggered by the presence of peptides derived from the prolamins fragmentation, which are not digested by gastrointestinal proteases and which are found to be toxic for celiac patients. Peptide structure is essential to produce the toxic effect, because when the polypeptides from the prolamins are hydrolyzed to the constituent amino acids, it loses the ability to damage the intestine. Among the "toxic peptides" identified, the so-called 33-mer (SEQ ID NO. 1, residues 57-89 of gliadin), containing 6 epitopes for T cell recognition, is one of the most immunogenic peptides present in gliadin and one of the responsible for the immune reaction that is triggered in the intestine of celiac patients (Shan et al., 2002, Science, 297: 2275:2279; Sollid, 2002, Nat Rev Immunol., 2:647-655). These authors demonstrated by *in vitro* and *in vivo* studies with rats and humans that the 33-mer peptide from α-gliadin is very resistant to gastric, pancreatic and intestinal enzymatic proteolysis. These peptides pass through the intestinal mucosa where they interact with tissue transglutaminase, which transforms glutamine residues into glutamic acid and, as a result, there are a number of changes that trigger reactions with an immunological basis that end up destroying the intestinal villi.

As noted above, currently the only effective treatment for CD is to maintain a strict diet free of products elaborated with wheat, barley, rye and possibly oats. The presence of this cereal in "gluten-free" food is controversial. Oats differs from other cereals because of its prolamins content, which represents 10-20% of total protein. Moreover prolamins from different cereals vary in molecular size and amino acid content. The proportion of proline and glutamine in avenin (amino acids abundant in the toxic regions) is lower than in other cereals. Some researchers claim that celiac patients tolerate oats without signs of intestinal inflammation; in fact the use of oats in "gluten-free" food is allowed in many countries, for example Gluten Free Oats®. The reactivity observed in oats with an antibody specific for gliadin has been attributed to contamination with wheat of foods based in oat (Hernando et al., 2008, Eur J Gastroenterol Hepatol., 20:545-554). In contrast, there are studies confirming the toxicity of oats in certain types of celiac patients and the impossibility of oat consumption regularly. Arentz-Hansen et al. (2004, PloS Medic., 1:84-92) described intestinal damage suffered by some patients after consumption of oats and a gluten-free diet. These patients may trigger an immune response against avenin similar to that produced by the gluten from wheat, rye or barley. A study led by Dr. Knut Lundin (Lundin et al., 2003, Gut, 52:1649-1652) with a total of 19 adults with celiac disease who were consuming 50 g of oats daily for twelve weeks showed that one of the celiac patients of the study proved to be sensitive to oats. Intestinal biopsy showed partial atrophy, which significantly recovered after stopping oats intake, but again developed subtotal atrophy and acute dermatitis after eating oats again. This suggests the need to distinguish between celiac groups according to their sensitivity to cereals and to identify the source of the immunogenicity of avenin peptides. Silano et al. (2007, J Gastroenterol Hepatol., 22:528-531; 2007, Scand J Gastroenterol., 42:1302-1305) carried out a series of *in vitro* assays with different varieties of oats, applying various immunological techniques including activation of peripheral lymphocytes of patients with celiac disease, and found that all varieties under study were toxic to celiac patients but there were differences in the levels of toxicity. Therefore, it is critical to clarify qualitatively and/or quantitatively the immunotoxic potential of oats for celiac patients because of the obvious clinical consequences.

Other authors (Spaenij-Dekking et al., 2005, Gastroenterology, 129:797-806) have suggested that there are variations in the toxicity of different wheat varieties, indicating the possible selection of nontoxic varieties for celiac patients by sequencing of the prolamins, T cells reactivity and the use of certain antibodies specific against different subunits from gliadins and glutenins. This paper does not propose a reliable and practical method to reproduce in all cases the results obtained by T cell reactivity, requiring even the use of multiple antibodies to have conclusive data, which makes it impractical for application in industry and complicates conclusions that can be obtained because of the differential in the immunotoxic potential of each peptide detected.

On the other hand, other authors are working on obtaining wheat seeds with reduced amounts of gliadin using gene suppression techniques (Gil-Humanes et al., 2010, Proc Natl Acad Sci USA, 107:17023-17028). Practical techniques that allow the selection and even the quantification of the potential toxicity of the seeds of various cereal lines selected will be needed.

To measure the potential toxicity for celiac people of seed varieties by assays with T cells isolated from celiac patients is an unfeasible technique to use it routinely in the industry because it requires working with peripheral blood of a number of celiac patients, very specialized staff, expensive reagents and optimal preservation of these immunological cells. On the other hand, it is needed that the experiments to perform are approved by the Ethics Committee of the Hospital where will be the sampling. With this background, the ideal would be to have an immunological *in vitro* assay that reflects as much as possible the information that can be obtained after conducting experiments of reactivity with T cells isolated from individuals with celiac disease, and compare it with the reactivity of antibodies obtained against some immunotoxic epitopes predominant in gluten present in the seeds. These epitopes recognized would have to be absent in certain grains such as corn or rice, and have its analogue in immunotoxic cereals (wheat, barley, rye), and present less reactive analogues in oats, reflecting the potential immunotoxicity of each variety. In previous studies, two monoclonal antibodies (moAbs), G12 and A1, against 33-mer peptide from gliadin were obtained (Morón et al., 2008, Am J Clin Nutr., 87:405-414). The results obtained showed that the reactivity of each moAb correlates with the immunotoxic potential of food cereals from which proteins were extracted. The immunological assays performed with these antibodies demonstrated that they are the only ones described in the market capable of recognizing 33-mer peptide with a high specificity (Morón et al., 2008, Am J Clin Nutr., 87:405-414; 2008, PloS One, 3:e2294; Cebolla et al., 2009, 13th International Coeliac Disease Symposium, Amsterdam, The Netherlands). 33-mer peptide is characterized because it contains 6 epitopes for T cell recognition. It is one of the most immunogenic peptides present in gliadin and one responsible for the immune reaction that is triggered in the intestine of celiac patients. The immunological assays performed with the G12 antibody demonstrated that it is the only one, besides moAb A1, described in the market capable of recognizing 33-mer peptide with a high specificity (Morón et al., 2008, Am J Clin Nutr., 87:405-414; 2008, PloS One, 3:e2294). Studies with T cells indicated that the activation of these cells by gluten digested with glutenases (enzymes that digest gluten peptides) decreased in an equivalent way to the detection of toxic peptides with the antibodies (Morón et al., 2008, PloS One, 3:e2294; Ehren et al., 2009, Plos One, 4:e6313). In addition, the immunological systems described are capable of detecting other immunogenic peptides that participate in the innate and adaptive immunity of celiac disease.

Studies with T cells indicated that the activation of these cells by gluten digested with glutenases (enzymes that digest gluten peptides) decreased in an equivalent way to the detection of toxic peptides with the antibodies (Morón et al., 2008, PloS One, 3:e2294; Ehren et al., 2009, Plos One, 4:e6313). This assay enables to avoid false negatives and positives that are obtained when using an analysis method that does not take into account the real toxicity of the gluten.

In these previous studies, the anti-33-mer from gliadin antibodies, A1 and G12, showed reactivity against oat amounts relatively higher than gliadin from wheat. The reactivity was clearly above negative control although its detection was less sensitive (Morón et al., 2008; PloS One, 3:e2294).

### DESCRIPTION OF THE INVENTION

The present invention describes a method that can be industrially applied to identify oat seeds and other grains that are tolerable and safe for celiac patients. It is based on simple immunological assays mediated by one or more monoclonal antibodies which detect the 33-mer peptide and other related peptides toxic to celiac people. The described method is able to detect epitopes which are sufficiently similar in oats to that previously described as the most immunotoxic in wheat, and are detectable in some varieties of oat and not in others. The intensity of the signal obtained with the anti-33-mer antibody is proportional to the potential damage caused to celiac subjects, measured by cell proliferation and production of interferon-gamma (INF-γ) by T cells from these patients. The method described in this invention is specific and reliable to detect oat varieties potentially safe for celiac patients.

Seeds that give no reactivity with the anti-gliadin 33-mer antibody would be safe to be consumed by celiac patients, could be cultivated and sold certified for their safety in the consumption by celiac people, since the only effective treatment to date for this disease is a gluten-free diet. These immunological methods would be preferably ELISAs, but also contemplate immunochromatographic strips, labeled fluorescent microparticles coated with antibodies (for example Xmap technology by Luminex), Western blots and biosensors with detection by antibodies. These methods consist of one or several monoclonal antibodies capable of identifying and quantifying the most immunogenic epitopes from gluten. The preferred antibody for its use in the seed selection would be anti-gliadin antibody G12, which has the recognition epitope SEQ ID No. 2 within the 33-mer described by Morón et al. (2008, PLoS One, 3:e2294). It also could serve as the monoclonal antibody A1 (recognition epitope SEQ ID No. 3 within the 33-mer), described by the same authors, also obtained by immunization with the 33-mer, and even R5, which although from 100 to 60,000 times less sensitive to detect the 33-mer than G12 and A1, detects other peptides similar to 33-mer.

A preferred form of the invention would be to perform a competitive ELISA type immunoassay wherein the monoclonal antibody is the moAb G12 which would be conjugated to an enzyme typically used in these methods such as alkaline phosphatase or peroxidase. It could be used in the assay gliadin, hydrolyzed gliadin or 33-mer peptide as standards.

The validity of the concept would be manifested by the proportionality relation between the signal obtained by reactivity with the anti-gliadin 33-mer monoclonal antibody, preferably G12, and the potential damage caused by toxic proteins in celiac individuals measured by cell proliferation and INF-γ production by T cells from patients with CD.

Another object of the invention is the application of a competitive ELISA, a sandwich ELISA or a Western blot with the G12 antibody characterized by its ability to detect prolamins from wheat, barley, rye, and, according to their immunogenic potential, oat prolamins.

This process is characterized by its ability to identify and quantify the most immunogenic gluten peptides in different varieties of a cereal, as well as for the selection of cereals used in the preparation of ingredients forming part of drug carriers, and other industrial applications in products to be ingested by celiac people.

Through studies of reactivity with anti-33-mer antibodies against the different varieties and their correlation with biological assays with T cells, it would be demonstrated that there is a high variability in the immunotoxic potential of different cultivars of oats.

Since T cells or similar biological assays are not routine techniques commonly used to evaluate the risk of food in food safety controls, immunochemical techniques based on antibodies that reflect said toxicity in a reproducible way are suitable methods to assess the potential immunotoxicity that a cereal given may have to the celiac people.

This invention provides a rational path towards the selection of varieties of cereals, particularly oats, that might be safe for a gluten-free diet, which would allow the incorporation of this cereal in the diet of celiac patients, giving a great benefit to this group, since in recent years the interest in oats for human consumption has increased due to the recognition of its nutritional value and health benefits. Oat grains contain high amounts of nutrients such as soluble fiber, proteins, unsaturated fatty acids, vitamins, minerals and phytochemicals. The incorporation of some varieties of oats and other cereals determined as safe by this method could be introduced in the gluten-free diet of celiac people, which would improve the quality of nutrition of celiac patients and allow its use in the diets of certain diseases. Another object of the present invention is the selection of other cereals by the same immunological method that can have high variability in immunotoxic peptides content, as wheat and other cereals seeds genetically manipulated in order to suppress immunotoxic peptides. This previous selection of seeds could be subsequently verified by immunological assays in cells from celiac patients.

### FIGURES DESCRIPTION

To complement the description that has being made and in order to help a better understanding of the characteristics of the invention, according to a preferred practical embodiment thereof, it is attached as an integral part of said description, with illustrative and non limited to, the following figures:

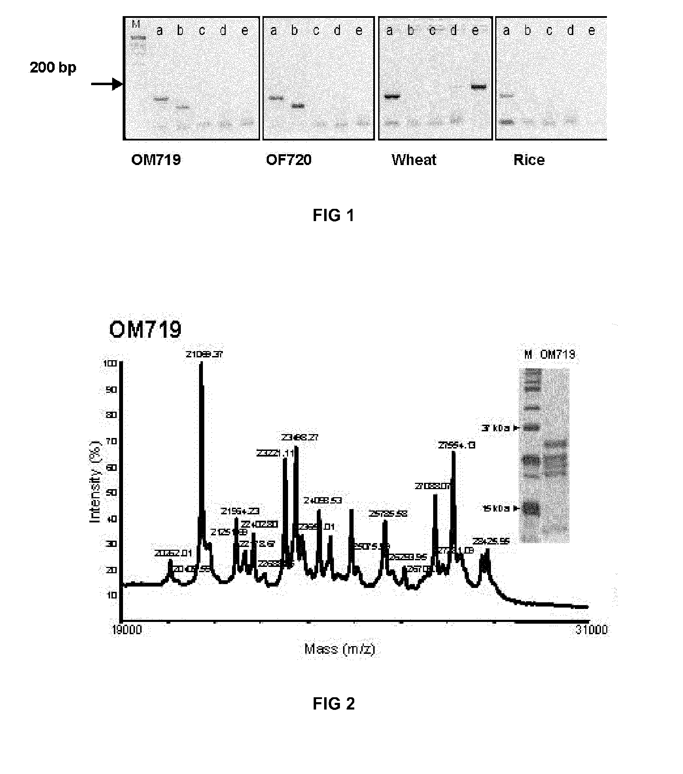

**Figure 1****.** Evaluation of the presence of amplifiable DNA in oat varieties, as well as in wheat and rice by PCR. Agarose gel of the PCR amplification products of two representative samples of oat, wheat and rice. M: molecular weight marker of DNA (200 bp). Positive control: cereals DNA amplified with primers 18S. The primers used were: a. 18S, b. ω-avenin (oats), c. ω-hordein (barley), d. ω-secalin (rye) and e. ω-gliadin (wheat).

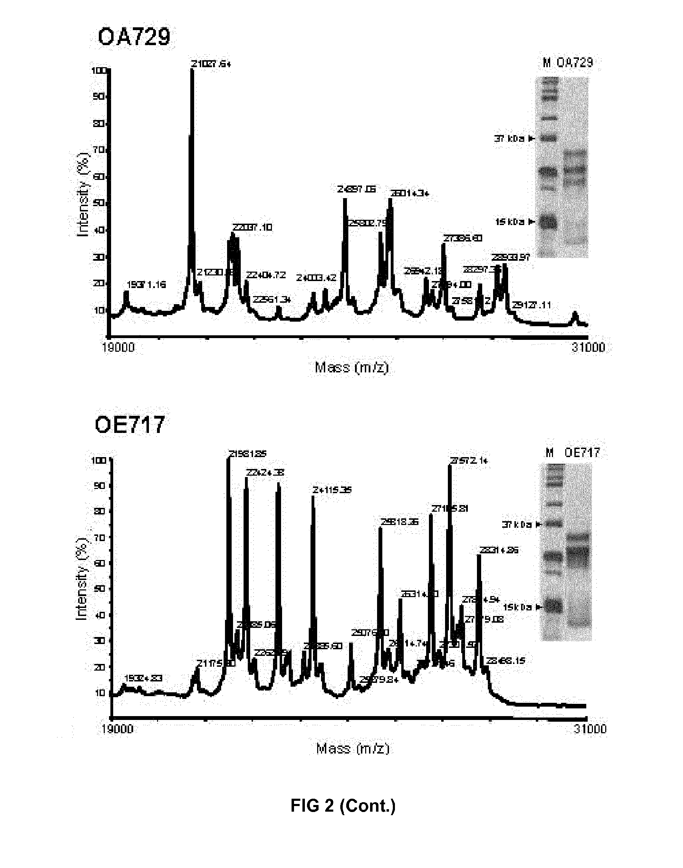

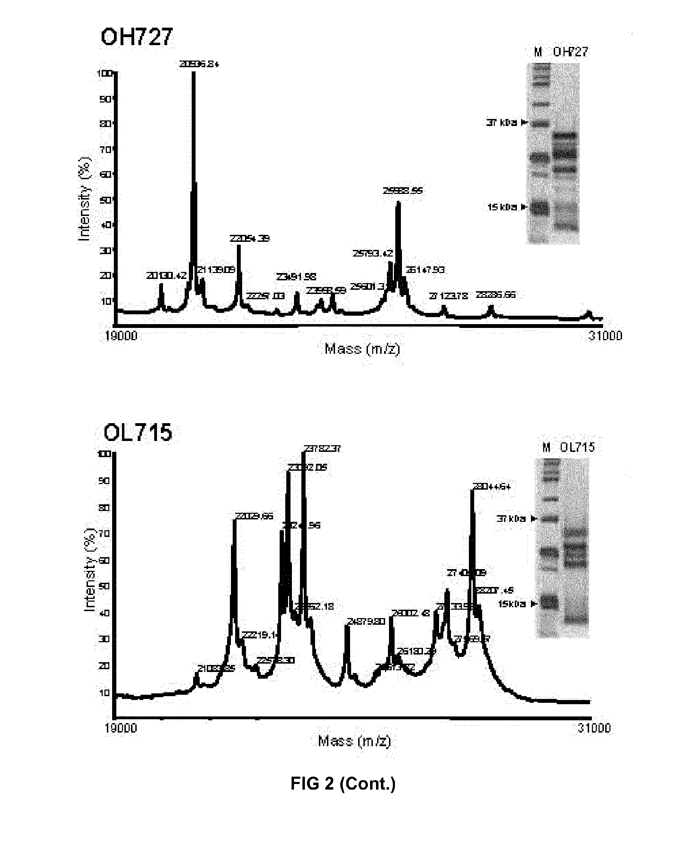

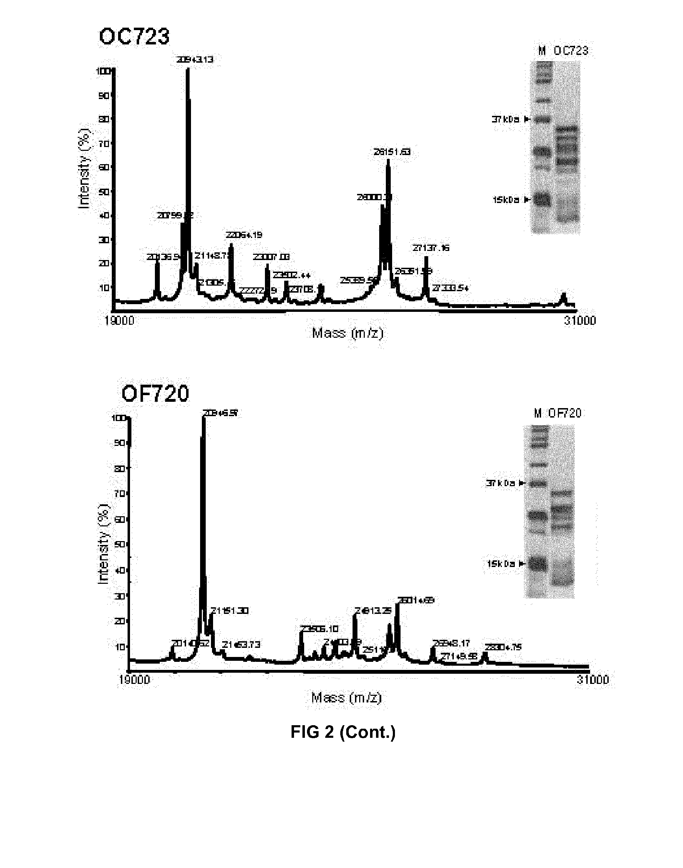

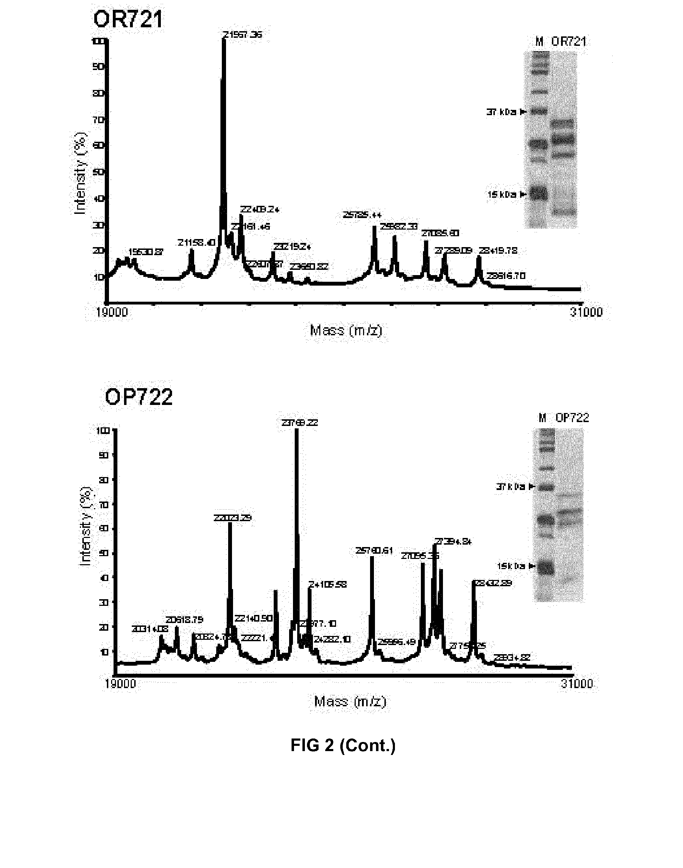

**Figure 2****.** Comparison of avenin fractions extracted from different varieties of oats. Avenins spectrum of 9 different varieties obtained by MALDI-TOF MS. Analysis of the protein extract from the different varieties by polyacrylamide gel electrophoresis. M: protein molecular weight marker.

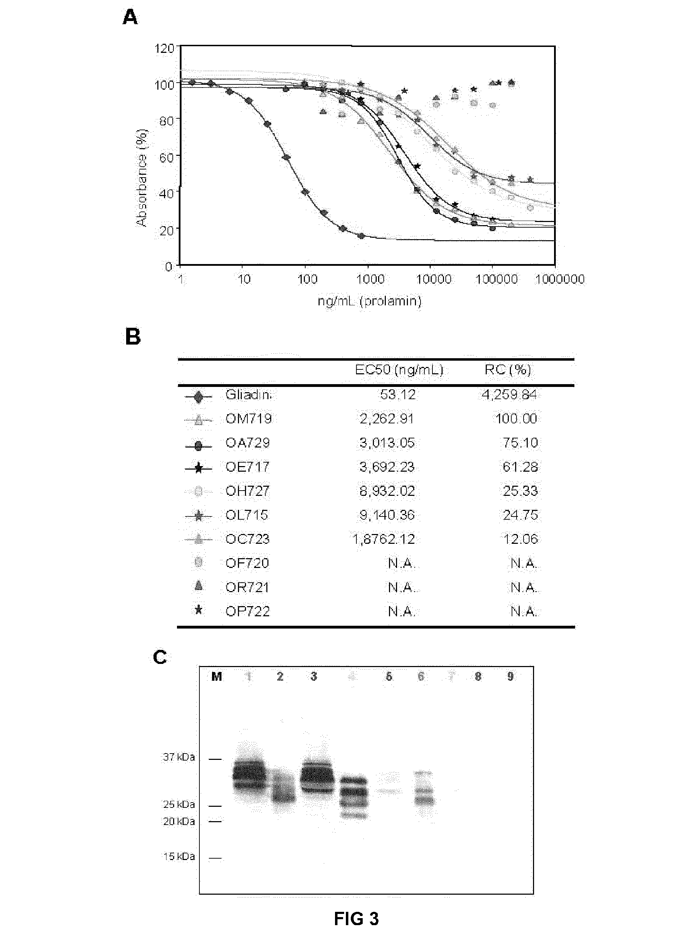

**Figure 3****.** Relative affinity of the monoclonal antibody G12 against different varieties of oats. A. Competitive ELISA using G12-HRP antibody to determine the reactivity of this antibody against different varieties of oats. Three tests were performed with three replicates each one. As a positive control gliadin was used. B. IC50 and RC of the different varieties of oats. N. A.: Not applicable. C. Western blot of the toxic fractions of prolamins extracted from oat grains. The membrane was revealed with the antibody G12. The color code used was the same than in A and B. M: protein molecular weight marker.

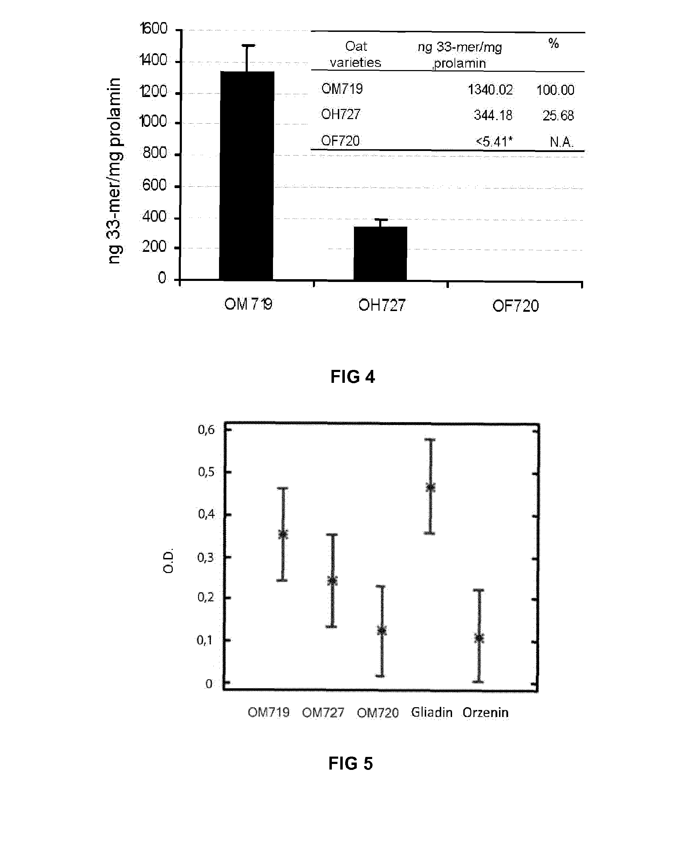

**Figure 4****.** Detection of the concentration of 33-mer peptide in different varieties of oats. The concentration of 33-mer was determined by competitive ELISA using the G12-HRP antibody. Three tests were performed with three replicates each one. Percentage of 33-mer of the variety tested compared to the variety more reactive, OM719. * 33-mer concentration below the limit of quantification of the assay (5.4 ng / mL). N. A.: Not applicable.
**Figure 5****.** Proliferative response of T cells from celiac patients against deamidated peptides of prolamins from three varieties of oats. PBMCs were exposed for 48 hours to prolamins previously digested and treated with tissue transglutaminase. Values are expressed as units of O.D. at 450 nm. Gliadin and orzenin were used as positive and negative control, respectively. Mean ± SEM is represented for two trials on two separate days. * Statistically significant differences regarding orzenin (p <0,05).

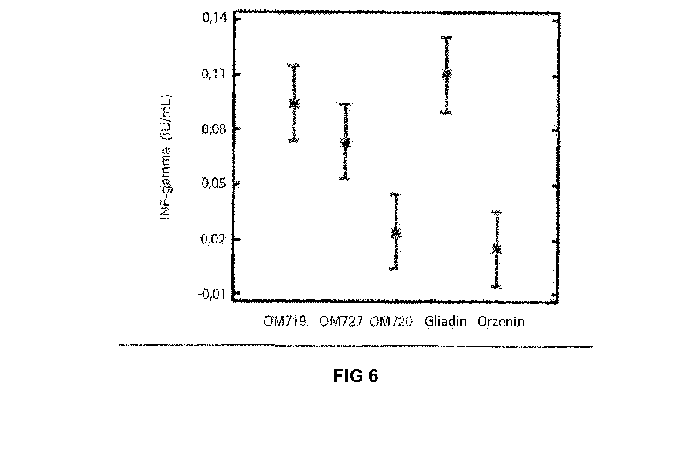

**Figure 6****.** INF-γ production by T cells from celiac patients exposed to previously digested and deamidated prolamins from three varieties of oats. The production of INF-γ was assessed by ELISA after 48 hours of incubation. Gliadin and orzenin were used as positive and negative control, respectively. Mean ± SEM is represented for two trials on two separate days. * Statistically significant differences regarding orzenin (p <0,05).

### PREFERRED EMBODIMENT OF THE INVENTION

### Example 1. Determination of the purity of various samples of oats

The present example shows the importance of checking the purity of oat seeds that are going to be analyzed before determining its immunotoxic potential, since any contamination of other cereals (barley, wheat, etc.) would give false positives. Previous work has suggested that the toxicity of foods elaborated with oats is due to contamination with other cereals toxic for celiac patients (wheat, barley and rye). An example of this is the work published by Hernando et al. (2008, Eur J Gastroenterol Hepatol., 20:545-554) which states that the R5 antibody reactivity against certain foods made with oats is due to contamination of this cereal with wheat, barley or rye (Pulido and al., 2009, Adv Food Nutr res, 57:235-285). For this study, the purity of the samples of different varieties of oats was controlled in two ways. The first one performing a visual examination of individual oat grains to avoid the presence of grains of other cereals. The second form of control was using the polymerase chain reaction (PCR) since it is a highly specific and sensitive method for detecting small amounts of nucleic acid. In food quality studies, PCR has been applied to the detection of genetically modified organisms, pathogenic fungi, microorganisms and viruses as well as for the identification of animal species in meat products or for the detection and discrimination of contamination with cereals in gluten-free food. In general, the presence of cereal proteins is related to the presence of DNA, in this study the PCR technology has been used to detect nucleic acid from various cereals (wheat, rye and barley) in the oat samples.

To detect contamination of cereals (wheat, rye and barley) in the different varieties of oats, specific target sequences of encoding fragments from gliadin (wheat), secalin (rye), hordein (barley) and avenin (oats) were chosen for the amplification. The nucleotide sequences of these cereal storage proteins genes are well characterized and sequenced, showing a high degree of homology between the sequences of the prolamins genes of wheat, barley and rye. However, it was possible to design pairs of primers that could discriminate between different cereals. The primers used for amplification of ω-gliadin, ω-secalin, ω-hordein and ω-avenin genes were designed with the software PRIMER3 (http://frodo.wi.mit.edu/cgi-bin/primer3/primer3 _www.cgi). The specific forward and reverse primers, respectively, were: 5'-TCTGCCCTATCAACTTTCGATGGTA-3 'and 5'-AATTTGCGCGCCTGCTGCCTTCCTT-3' for the 18S ribosomal gene, 5'-CAGAAAGCGAGTGGAAAGATGAAAG-3 'and 5'-GCAAGGAGGACAAAGA TGAGGGAA-3' for the gene ω-gliadin (amplified fragment length of 181 bp), 5'-TTTTTCAGAAAGCGAGTTCAATGATG-3 'and 5'-CGAGGACAAAGATGAGGAAGGTCT-3' for the ω-secalin gene (amplified fragment length of 181 bp), 5'-ATTAATTCCCAAACTGAACGACTA-3 'and 5'-CATGGCGAACAATGTGAAC-3' for the ω-hordein gene (amplified fragment length of 164 bp), 5'-CGCTCAGTGGCTTCTAAGA-3 'and 5'-TTTTATTTTATTTGTCACCGCTAC-3' for ω-avenin gene (amplified fragment length of 104 bp). The oligonucleotides used were provided by Biomedal S.L. (Seville, Spain).

PCR system was used for the amplification of prolamins genes from wheat, barley, rye and oats. To do this, seeds of oat (*Avena sativa*) from different varieties: OM719, OE717, OL715, OA729, OH727, OC723, OF720, OR721 and OP722, supplied by different Spanish and Australian commercial sources were used. Wheat (*Triticum durum,* variety Don Pedro, CSIC, Córdoba, Spain) and rice (Oryza *sativa* subspecies Japonica, variety J. Sendra, Rice Federation, Seville, Spain) seeds were used as positive and negative control, respectively.

Grains from different cereals were ground on an electric grinder to obtain homogeneous samples. Nucleic acid extraction was performed using the modified CTAB method. Each sample was frozen with liquid nitrogen and transferred to a 2 mL tube containing 1 mL of extraction buffer [Tris-HCl 100 mM (pH 8.0), NaCl 1.4 M, EDTA 1.4 M (pH 8.0), CTAB 2% (w/v) and 2-mercapto-ethanol (14.3 N)]. Subsequently, samples were incubated at 65 °C for 30 minutes shaking periodically. After incubation, 500 µL of chloroform were added and samples were centrifuged at 16,000 g for 15 minutes. The supernatant was transferred to a new 2 mL tube, to which 0.8 volumes of isopropanol were added to precipitate the DNA. The washes were performed with ethanol 70% (v/v). Finally, DNA was eluted in 60 mL of Tris-HCl 10 mmol/L (pH 8.0) containing 20 µL/mL of RNase (RNase A Solution, Promega). DNA concentrations were determined by UV absorption at 260 nm and purity of the DNA solution was evaluated by absorption ratio 260/280 nm.

PCR premix (Biotools B&M Labs, Madrid, Spain) was used with 1 pmol of each primer and 50 ng of DNA template. The amplification reaction was performed under the following conditions: cycle 1, 95 ° C for 3 min; cycle 2, 95 ° C for 30 s, 52 ° C for 30 s and 72 ° C for 30 s (repeating the same conditions for 35 cycles); cycle 3, 72 ° C for 5 min. PCR products were analyzed by gel electrophoresis at 2% (w/v) agarose MS (Roche Diagnostics, Mannheim, Germany).

In this way it was determined that the amplified fragment length varied between the different cereals from 104 bp for oats, 164 bp for barley up to 181 bp for wheat and rye. In these experiments, negative results were obtained in oat samples for specific wheat, rye and barley PCR; however, positive results were obtained by amplifying oat samples with specific primers 18S. Wheat amplification showed positive results in the specific PCR for 18S and gliadin. The agarose gel analysis of the DNA products amplified by PCR confirmed that all oat samples were free of contamination with wheat, barley, rye or a mixture of these cereals (Figure 1).

### Example 2. Variability analysis of the avenins of oats cultivars

The present example of the application shows that there is a large variability of avenins from different oats cultivars, which in principle is also indicative of a potential diversity in potential immunotoxicity. MALDI-TOF MS technology (matrix-assisted laser desorption/ionization time-of-flight mass spectrometry) is currently used for the identification of a large number of subunits of glutenin and gliadin in wheat and other cereals (Qian et al., 2008, J Am Mass Spectrom., 19:1542-1550). In this study we have used this technique together with SDS-PAGE (polyacrylamide gel electrophoresis with sodium dodecyl sulfate) to analyze the content in oat prolamins and to find molecular features that allow the identification and characterization of the different varieties. To do this, flour samples (6 g) of the different varieties of oats were obtained by crushing the seeds, resuspended in 70% ethanol (v/v) (30 mL) and maintained for 24 hours under stirring. After that, the mixture was filtered and prolamins were precipitated by addition of absolute ethanol to obtain a final concentration of 93%. Prolamins were obtained after incubation of the samples at 4 ° C for 16 h. After this time it was centrifuged at 8,000 g for 10 minutes and the precipitate containing the proteins under study was collected. The protein concentration was measured by the Bradford method (Bradford, 1976, Anal Biochem., 72:248-254).

For the analysis of the avenins extractions by MALDI-TOF MS (5 µL) 2 µL of detergent octyl-β-D-glucopyranoside was added, and 25 µL of saturated sinapinic acid in 30% acetonitrile (v/v) containing 0.1% trifluoroacetic acid (v/v) was used as matrix solution. Sample-matrix mixture was dried for 15 min in a speed-vac centrifuge and then resuspended in 6 µL of 60% ethanol with 0.1% trifluoroacetic acid. 2 µL of the mixture was placed on a stainless steel probe and dried for 5 min at room temperature. The samples were measured in the PE Biosystems MALDI-TOF Voyager DE-PRO system with a standard configuration. The mass spectrum was recorded in positive linear mode with an acceleration voltage of 25 kV and the final spectrum was obtained by accumulating 200 spectra of single laser shot under irradiation threshold. The equipment was externally calibrated using single and double BSA load signals with molecular masses of 66,430 and 33,215 Da respectively. Identification of avenins by MALDI-TOF MS showed characteristic profiles of protonated masses (Camafeita et al., 1977, J Mass Spectrom., 32:444-449).

For the analysis by SDS-PAGE, prolamins previously extracted were diluted in running buffer (Tris-HCl 62.5 mM at pH 6.8, 10% glycerol, 2% SDS, 0.001% bromophenol blue and 5% 2-mercapto-ethanol) and denatured by boiling at 100 °C for 5 min This step was repeated a total of three times. Samples were run on 12.5% polyacrylamide gel at a constant voltage of 100 V using MiniProtein system (BioRad Laboratories). The separated proteins in the gel electrophoresis were stained using silver staining.

The spectrum of avenins fractions from different varieties of oats obtained by MALDI-TOF MS, as well as the electrophoretic distribution of the prolamins observed by SDS-PAGE, disclosed that different varieties showed different protein profiles, which could allow a more comprehensive identification (Figure 2). Both in the spectrum obtained by MALDI-TOF as by SDS-PAGE, in all cases, the molecular weight distribution was located between 19 and 31 kDa, which agrees with that described to date by others (Chesnut et al ., 1989, The Plant Cell, 1:913-924, Hernando et al., 2008, Eur J Gastroenterol Hepatol., 20:545-554). The number and relative intensity of the peaks obtained by MALDI-TOF in the 9 cultivars was highly variable, which indicates that differences exist between the avenins extracted from different cultivars. The variability in size and protein distribution was also evident in SDS gels; the bands obtained differ in electrophoretic mobility and intensity. These results indicate the presence of different subunits of prolamins in the 9 oat varieties which differ in their amino acid composition and size.

### Example 3: Differential reactivity of an anti-gliadin 33-mer antibody against different oat varieties

This example demonstrates how an antibody that recognizes the peptide 33-mer of gliadin, can have a great variability in reactivity with the different varieties of oats. The example shows how the G12 moAb that was able to detect epitopes related to the 33-mer peptide in several cereal prolamins, reacts differently with different varieties of oats being its reactivity null in some of these varieties. The results indicate that the anti 33-mer moAb shows reactivity against prolamins of wheat, barley and rye, cereals toxic for celiac patients. This antibody was also able to detect avenins present in oats, although the sensitivity obtained in this case was lower, which may be due in part to the smaller proportion of these prolamins with respect to total protein content of this cereal in relation to the proportion of gliadins, hordeins or secalins in their respective grains, and mainly to the lower affinity of this antibody to epitopes of the avenins. G12 antibody did not react with prolamins extracted from rice (orzenin) and corn (zein), cereals that are not toxic for celiac patients. The reduced sensitivity of the G12 antibody against oat prolamins could be due to a lower prevalence of recognition epitopes of the antibody in the avenins with respect to the gliadins, hordeins and secalins.

To determine whether the moAb G12 showed different reactivity to different varieties of oats, the affinity of the antibody was determined by competitive ELISA. For this assay Maxisorp microtiter plates were used (Nunc, Roskilde, Denmark), which were coated with 100 µL/well of gliadin solution Sigma (5 µg/mL) in 0.1 M carbonate buffer (Na₂C0₃-NaHC0₃, pH 9.6), and incubated at 4°C overnight. The plates were washed with PBS 0.05% Tween ® 20 and blocked with blocking solution (PBS, 5% skimmed milk) for 1 h at room temperature. Serial dilutions of gliadin and study samples were made in PBS with 3% BSA (100 µL) and 100 µL of moAb G12 conjugated to HRP (peroxidase) solution was added to each one (1:10,000 in PBS with 3% BSA). Samples were pre-incubated 1 h at room temperature with gentle agitation, and then added to the wells. After 30 minutes of incubation, samples were washed, and 100 µL/well of substrate solution (TMB, Sigma, St Louis, Missouri, USA) was added. After 30 minutes incubation at room temperature in darkness, the reaction was stopped with 1 M sulfuric acid (100 µL/well) and the absorbance was measured at 450 nm (UVM340 microplate reader, Asys Hitech GmbH Eugendorf, Austria).

The results obtained showed that different oat varieties presented different reactivity to the G12 antibody (Figure 3A). Depending on the affinity of the antibody to the different oat varieties, three groups could be clearly distinguished: a group with high affinity to the antibody (OM719, OA729 and OE717 varieties), a group with an intermediate recognition (OH727, OL715 and OC723 varieties) and another that was not recognized by the G12 moAb (OF720, OR721 and OP722 varieties). Results similar to those described were obtained with antibody A1 (Morón et al., 2008, PLoS One, 3: e2294).

In order to quantify the affinity of the antibody G12 for the different varieties of oats, it was determined the antigen concentration with which a reduction of 50% of the maximum signal was obtained (IC50) and cross-reactivity (CR) of each one of them (Figure 3B). CR was determined as (IC50 of the oat variety that present the highest affinity for the antibody/IC50 of each variety tested) x 100. OE717 and OA729 varieties showed a CR around 60 and 75% respectively, compared to the most sensitive variety (OM719). Additionally, OH727, OL715 and OC723 with a CR of 25, 24 and 12% respectively, are recognized by the antibody G12 but with less sensitivity. As with negative control (rice), the avenins from varieties OF720, OR721 and OP722 were not recognized by the antibody. The results obtained suggest that OF720, OR721 and OP722 oat varieties could potentially be safe for use in a gluten-free diet in celiac patients.

With the aim of confirming the results of the competitive ELISA using another immunological technique and identifying reactive protein profile for the anti-33-mer antibody, an assay was performed by immunoblotting using moAb G12. Initially obtained protein extracts were separated by SDS-PAGE gel and then incubated with antibody G12 on PVDF membranes. The extracts were incubated in blocking buffer (TBS with 5% skim milk) overnight and after that G12 antibody was added (dilution 1: 5000 in blocking solution). After 3 washes, membranes were incubated with secondary antibody anti-mouse IgG conjugated to phosphatase (Sigma, St. Louis, MO) (dilution 1: 2000 in blocking solution). The membrane was developed using Sigma-Fast system. The results obtained by Western blot (Figure 3C) showed that moAb G12 has affinity for OM719, OA729, OE717, OH727, OL715 and OC723 varieties. However, this antibody did not react with OF720, OP722 and OR721. Thus, the variability in the reactivity observed in Western blot analysis confirms the results previously obtained in the competitive ELISA, and it was observed that the protein profiles obtained from reactive varieties differ from each other. OM719 and OE717 varieties presented similar reactive protein fractions; however, OA729, OC723, OL715 and OH727 presented protein profiles which bands differing in molecular weight and intensity. These results suggest the presence of different subunits of prolamins in oat varieties, which differ from each other in terms of amino acid composition and size. Likewise, the differences in reactivity with the monoclonal antibody G12 suggest variations in the protein sequence of the antibody recognition epitopes present in the avenins of different oats varieties.

### Example 4: Determination of the effective amount of immunoreactive peptides in oats

This example shows how the equivalent concentration of 33-mer peptide in seeds can be determined using an anti-gliadin 33-mer antibody with a peptide with this sequence as a reference. The 33-mer peptide has been identified as one of the main contributors to the immunotoxicity of gluten (Shan et al., 2002, Science, 297:2275-2279). This peptide from the α-gliadin contains 6 recognition epitopes for T cells and is very resistant to proteolysis. G12 monoclonal antibody is specific for the epitope of 6 amino acids SEQ ID No. 2, which appears 3 times along the peptide sequence of the 33-mer. This monoclonal antibody is able to recognize other immunoreactive peptides present in the gliadin and other toxic prolamins (Morón et al., 2008, PloS One, 3:e2294; Ehren et al., 2009, PLoS One, 4:e6313).

In order to determine the relative amount of immunotoxic epitopes present in prolamins of different oat varieties, we chose a variety from each group previously identified. For the group with greatest affinity for the antibody we chose OM719, for intermediate reactivity group, we chose the variety OH727, and OF720 from the group not recognized by G12 antibody. The presence of immunoreactive peptides was determined by competitive ELISA G12, using standard peptide 33-mer. The assay was carried out using the same ELISA protocol described above, but in this case 33-mer peptide was used as a standard curve to measure the concentration of toxic epitopes in the samples studied. The concentration of 33-mer in each sample was determined by calculating the equation defined by the standard curve and subsequent extrapolation from the absorbance data obtained for each sample.

Equivalent presence of 33-mer in the more reactive variety, OM719, was approximately 1.340 ng per mg of avenine (Figure 4). In the OH727 variety the levels of 33-mer were diminished in the order of 4 times with respect to those of OM719. However, in the case of OF720, the concentration of 33-mer peptide was reduced more than 1300-fold relative to OM719, reaching undetectable levels by our method. These results confirm the data obtained previously by IC50 and cross-reactivity, as well as by Western blot. Likewise, these results indicate the high difference existing between varieties with regarding the presence of immunotoxic sequences for celiac patient and the possibility of finding oat seeds without amounts of detectable 33-mer equivalents.

### Example 5. Correlation between G12 antibody reactivity and immunogenicity of the different oats varieties

The present example shows how changes in reactivity of the anti-33-mer antibody observed for the different oats varieties are correlated with the degree of immunogenicity of the cereal. Immunogenicity was determined by assays of T-cell proliferation and production of INF-γ. Avenins ability to induce the immune response was analyzed using peripheral blood lymphocytes of individuals with celiac disease, using wheat gliadin as positive control and rice prolamins as negative control. For this example, three cultivars of oats were selected, one of each group previously identified; thus the variety OM719 represented the group that showed greater affinity for the antibody G12, OH727 the intermediate reactivity group, and OF720 the group that was not recognized by the antibody. It was evaluated whether there was a correlation between immunotoxic potential of the different oats varieties and its reactivity against the antibody G12. To do this the following procedures were carried out:
5.1. Digestion with pepsin, trypsin and chymotrypsin. The ethanol soluble protein fraction was extracted from the flour and subjected to sequential digestion with pepsin, trypsin and chymotrypsin (Sigma, St. Louis, Missouri, USA). Gastrointestinal digestion was simulated so that the prolamins samples were incubated with 0.6 mg/ml of pepsin in 0.2 M HCl for 1 hour at 37°C with gentle agitation. After that, pH of the samples was modified with Na₂HPO₄ to pH around 6 to 7, then trypsin and chymotrypsin enzymes were added at a concentration of 0.375 mg/mL. The samples were again incubated for 30 minutes at 37°C with gentle agitation. For the inactivation of enzymes, the samples were heat treated (100°C for at least 5 min). Digested prolamins were lyophilized and stored at -20 °C until use.
5.2. Deamidation of samples with tissue transglutaminase: specific deamidation of prolamins peptides by tissue transglutaminase is usually necessary for the binding of antigen presenting cells to major histocompatibility complex proteins (DQ2/DQ8) and the subsequent recognition by T cells. Therefore, the avenins peptides from the different oats varieties, as well as gliadin and orzenin controls, were treated for 4 h at 37°C, with 100 g/ml of tissue transglutaminase (Sigma, St. Louis, Missouri , USA) in the presence of CaCl₂ 2 mM.
5.3. Patients: this study was performed after approval by the Ethics Committee of the "Hospital Virgen de las Nieves". Small bowel biopsies were obtained by gastrointestinal endoscopy under patients' informed consent for use in research on celiac disease (patients were monitored in the "Hospital Virgen de las Nieves" in Granada, Spain). Celiac disease patients showed partial or total atrophy of the villi with increase in intraepithelial lymphocytes. Histology positive cases were classified according to Marsh criteria (type I-IV) (Marsh and Crowe, 1995, Baillieres Clin Gastroenterol., 9:273-293).

The diagnosis of celiac disease patients was performed by serological test accompanied by intestinal biopsy and confirmation of a clinical response to the exclusion of gluten from the diet. Endomysial antibodies, anti-gliadin antibodies, anti-tissue transglutaminase antibodies and HLA typing specific for celiac disease (HLA-DQ) were determined.
5.4. PBMCs and cell culture: peripheral blood mononuclear cells (PBMCs) from patients with active celiac disease who follow a gluten containing diet, were isolated from 6 mL of heparinized blood by gradient centrifugation with HISTOPAQUE (Sigma, St. Louis, Missouri , USA) and subsequently grown to obtain a density of 1x10⁶ cells/mL in RPMI-1640 growth medium. After 48 h, the PBMCs were incubated with peptides derived from avenin, gliadin and orzenin (50 µg/mL).
5.5. Cell proliferation assay: T-cell proliferation was analyzed after 48 hours of incubation using the 5-bromo-2-deoxyuridine ELISA test (BrdU) for cell proliferation (Roche, USA). The results were expressed as optical density (O.D.) at 450 nm. Cell proliferation was determined by BrdU incorporation. This colorimetric test consists in the immunochemical measure of BrdU incorporation, an analogue of thymidine, to a DNA chain being elongated during active synthesis of this nucleic acid.
5.6. IFN-γ production: aliquots of the culture supernatant of T cells were taken after 48 h of incubation for the determination of the production of INF-γ, and stored at-80°C. A commercial ELISA kit was used for the analysis following the manufacturer's instructions (Thermo Scientific, Spain). Assay sensitivity was less than 2 pg/mL.
5.7. Statistical analysis of the cell proliferation assays and production of INF-γ: each experiment was performed in duplicate on different days. Data are expressed as mean ± standard error of the mean (SEM). All statistical analysis were performed using the program STATGRAPHICS for Windows. When the interaction was significant, differences between groups were assessed by ANOVA test. To compare individual means Bonferroni test was used corrected with Student's t. A statistical probability of p <0.05 was considered significant.

PBMC proliferation exposed to different avenins, and the positive and negative controls, gliadin and rice prolamins respectively, was expressed in O.D. (Figure 5). Thus, after 48 hours of exposure to the different avenins fragments, higher proliferation of T cells is found in the cultures incubated with the variety OM719 and gliadin (O.D.=0.35 and 0.47 respectively), and also with OH727 (O.D.=0.24). Thus, it is clear that gliadin and OM719 showed a higher reactivity being those with higher immunogenic potential (Figure 5). Incubation with the variety OF720 showed increased cell proliferation (O.D=0.12) similar to the negative control (orzenin peptides) (O.D.=0.11).

The INF-γ is the main cytokine involved in the inflammatory response of celiac disease. For that reason the secretion of IFN-γ was analyzed in the culture medium after exposure of peripheral T lymphocytes to deaminated avenin peptides (Figure 6). According to this analysis, gliadin and the variety OM719, were highly immunogenic, with higher values of secretion of INF-γ (0.11 IU/mL and 0.09 IU/mL, respectively), while exposure to OH727 induced a lower secretion of INF-γ (0.07 IU / mL). Finally OF720 and rice were less immunogenic (0.024 IU/mL and 0.015 IU/mL, respectively).

### Example 6. Potential immunotoxic detection with cereals with other antibodies with cross-reactivity against the 33-mer.

This example demonstrates how other antibodies which have not been created directly by immunization against 33-mer but which recognize it, although with less specificity, can be used to assess whether the oat seeds may be immunogenic for celiac patients. Although previous work established that R5 antibody reactivity against certain foods made with oats is due to contamination with wheat, barley or rye, and not to a direct recognition of epitopes of this antibody in oat prolamins (Pulido et al ., 2009, Adv Food Nutr Res 57:235-285), experiments performed for the present invention demonstrate R5 antibody reactivity with oat seeds with molecularly controlled purity. Following the instructions of the supplier, it was determined the capacity of moAb R5, specific antibody for the sequence QQPFP, to detect oat varieties previously tested. Similarly, the purity of the samples was monitored by PCR in order to avoid false positives.

The QQPFP peptide values obtained in OM719, OA729, OE717 and OH727 varieties ranged between 25.18 and 97.86 mg peptide/g cereal, which correspond according to supplier instructions to gliadin values between 100 and 400 ppm. In OL715 and OC723 varieties the peptide levels were between 1.5 and 9.2 mg peptide/g cereal respectively (6.3 to 37.2 ppm of gliadin, respectively). Thus the results obtained demonstrate that as the moAb G12 and A1, R5 antibody was able to detect the varieties described. However, in OF720, OR721 Y OP722 varieties gliadin was not detected. These results are equivalent to those already obtained by competitive ELISA G12. The detection of QQPFP like peptides in 6 oat varieties of 9 tested shows that the signal obtained by the R5 antibody in oat samples is due to the recognition of epitopes potentially toxic in prolamins of this cereal, and not to a cross-contamination with other cereals.

### Example 7. Detection of peptides equivalent to 33-mer in genetically engineered wheat seed

This example shows how in cereal seed genetically engineered to suppress the levels of the toxic gliadin, the level of reactivity with anti-33-mer antibody dicrease to a proportional extent to the immunogenicity. The transgenic lines published by Gil-Humanes et al. (2010, Proc Natl Acad Sci USA, 107:17023-17028) were tested with the monoclonal antibody G12 determining the concentration of toxic peptides present in each of these lines with respect to the controls.

Transgenic lines 28A, 28B, D783, X387, D770, D793, D894 and X077, transformed with vector pDhp-ω/α; E35, transformed with vector pDhp-ω/α + pghpg8.1; A 1152 and C655 transformed with the pghpg8.1 vector were analyzed with respect to control BW208. The toxic peptide 33-mer concentration was determined by competitive ELISA G12. The immunological assay showed a significant reduction of toxic peptide levels above 90% in the lines 28A, 28B, D783, X387, D770, D793 and X077. In contrast, D894 and E35 lines showed a reduction of 42.42 and 48.57 %. Meanwhile, the lines transformed with the vector pghpg8.1 showed no significant reduction in the levels of 33-mer with respect to the line BW208.

Other transgenic lines were also analyzed as D874, D876, X678 (transformed with vector pDhp-ω/α), C217 and D598 (transformed with vector pghpg8.1) with respect to control BW2003. D874, D876 and X678 present a reduction greater than 95%. However, in lines C217 and D598 the reduction percentage of toxic peptide was 52.21 and 43.96%, respectively.

This example again shows a correlation between toxic peptide levels obtained with the G12 antibody and T-cell results published by Gil-Humanes et al. (2010, Proc Natl Acad Sci USA, 107: 17023-17028) in cancelled gliadins from the transgenic lines used.

## Claims

1. A method for cereal seed selection **characterized by** the use of immunological methods with antibodies that recognize the 33-mer peptide from gliadin so that when they do not exhibit reactivity to gluten extracted from the seeds indicate that are tolerable by celiac patients.

2. A method for seed selection according to claim 1 wherein the immunological methods are indirect ELISA, competitive ELISA, sandwich ELISA, immunochromatographic strips, fluorescent immunomicroparticles, Western blots, biosensors, using at least one antibody that recognizes the 33-mer peptide of the gliadin.

3. A method for seed selection according to claim 1 wherein the immunological methods use at least one monoclonal antibody with ability to detect epitopes contained in the 33-mer of the gliadin SEQ ID No. 1, SEQ ID No. 2, SEQ ID No. 3, SEQ ID No. 4, SEQ ID No. 5, SEQ ID No. 6, SEQ ID No. 7, SEQ ID No. 8.

4. A method for seed selection according to claim 1 in which the immunological methods use at least one of the following monoclonal antibody G12, A1 and R5.

5. A method for seed selection according to claim 1 in which the immunological methods use the monoclonal antibody G12 conjugated to an enzyme that allows a quantitative assay using chromogenic, fluorogenic or luminescent substrates.

6. A method for seed selection according to claim 1 by an immunological assay according to claims 2-5, **characterized in that** the assay may use a gliadin pattern, gliadin hydrolyzate, whole 33-mer peptide or part of its sequence of at least 6 amino acids (SEQ ID No. 2).

7. A method for seed selection according to claim 1 in which the immunological methods are **characterized by** the proportionality between the signal obtained with the method object of the patent and the potential damage caused by the toxic proteins in celiac individuals measured by cell proliferation and production of IFN-γ by T cells from celiac patients.

8. A method for seed selection according to claim 1 using immunological methods according to claims 2-7 in which the seeds are wheat, barley, rye or oats.

9. A method for seed selection according to claim 1 using immunological methods according to claims 2-7 in which the seeds are oats.

10. A method for cereal cultivar selection according to claim 1 using immunological methods according to claims 2-7 in which the seeds are from genetically modified cereals to reduce their immunogenicity degree for celiac patients.

11. Use of cereal varieties selected according to claims 1 to 7 as an ingredient in foods which will be labeled as gluten-free.

12. Use of oat varieties selected according to claims 1 to 7 as an ingredient in foods which will be labeled as gluten-free.


SEQUENCE LISTING

<110> I. Comino, A. Real, C. Sousa
<120> Procedure for the selection of suitable cereal seed for consumption by
celiac patients
<160> 8
<210> 1
<211> 33
<212> PRT
<213> Triticum sp.
<400> Lys Gln Lys Gln Pro Phe Pro Gln Pro Gln Lys Pro Tyr Pro Gln Pro
      1               5                   10                  15

      Gln Lys Pro Tyr Pro Gln Pro Gln Lys Pro Tyr Pro Gln Pro Gln Pro
                  20                  25                  30

      Phe


<210> 2
<211> 6
<212> PRT
<213> Triticum sp.
<400> Gln Pro Gln Lys Pro Tyr
      1               5


<210> 3
<211> 7
<212> PRT
<213> Triticum sp.
<400> Gln Lys Pro Tyr Pro Gln Pro
      1               5


<210> 4
<211> 6
<212> PRT
<213> Hordeum sp.
<400> Gln Pro Gln Lys Pro Phe
      1               5


<210> 5
<211> 6
<212> PRT
<213> Triticum sp.
<400> Gln Pro Gln Lys Pro Lys
      1               5


<210> 6
<211> 7
<212> PRT
<213> Hordeum sp.; Secale sp.
<400> Gln Gln Pro Phe Pro Gln Pro
      1               5


<210> 7
<211> 7
<212> PRT
<213> Hordeum sp.

<400> Gln Lys Pro Phe Pro Gln Pro
      1               5

<210> 8
<211> 7
<212> PRT
<213> Triticum sp.
<400> Gln Gln Pro Tyr Pro Gln Pro
      1               5

1